# EUROPEAN PATENT APPLICATION

(11) **EP 1 231 274 A2**
(43) Date of publication of application: **14.08.2002**
(21) Application number: 02004082.0
(22) Date of filing: 20.11.1995
(51) Int. Cl.: C12N 15/62, C07K 14/81, C12N 5/10, A61K 38/55, A01H 5/00

(54) **Modified porteinase inhibitors**

(30) Priority: 21.11.1994 GB 9423477; 21.11.1994 GB 9423450
(62) Divisional of application: 95937951.2
(71) Applicant: THE UNIVERSITY OF LEEDS, Leeds, LS2 9JT (GB)
(72) Inventor: Atkinson, Howard John, Leeds LS8 1BY (GB); Mcpherson, Michael John, Dewsbury WF12 OLN (GB); Urwin, Peter Edward, Bramley, Leeds LS13 4NF (GB)
(74) Representative: Harrison Goddard Foote

(57) **Abstract**

The invention relates to proteinase inhibitors and in particular cystatins which have been modified so as to enhance their effective or synthetically manufactured counterpatrts. The modification including either site directed alterations in the structure of the protein and/or the production of hybrid molecules.

## Description

The invention relates to proteinase inhibitors and in particular novel proteinase inhibitors, methods for producing such inhibitors and products and processors including such inhibitors.

Proteinases are enzymes that break down proteins, their substrate specificity varies considerably and therefore does not form a basis for the purpose of classification. Rather, typically, these enzymes are classified according to the nature of the catalytic reaction that each undertakes. Thus proteinases are divided into four groups termed serine proteinases, cysteine proteinases, aspartic proteinases and metalloproteinases. Serine proteinases and cysteine proteinases are both widespread and diverse and are found in both prokaryotic and eukaryotic organisms, including plants and animals. In contrast, aspartic proteinases seem to be found only in eukaryotic organisms. Since these enzymes are used to break down protein the origin and/or the location of the enzymes determines whether they are beneficial or detrimental to a given organism. For example, where the enzymes are used by pathogens or parasites or pests they are typically used to break down host cell tissue and are therefore detrimental.

Pathogens, parasites or pests such as bacteria, fungi, plants, insects, nematode worms etc produce proteinases which break down host cell tissue to the detriment of the host.

For example, annual global crop losses caused by fungi exceed a thousand million pounds. The pathogen, *Botrytis cinerea* is of major economic importance because it causes disease in thirty crop plant species, with serious losses incurred in the glass house, in viticulture and as a result of post-harvest disease of fruit and vegetables. This major pathogen can be overcome with fungicides but unfortunately, there are disadvantages associated with the use of fungicides in order to control it. These include a financial burden associated with use of the fungicide, the potential environmental hazard arising from the use of toxic fungicides, with attendant consumer concern, and major problems of pathogen resistance to fungicides. In addition, many fungicides are effective against only a limited range of pathogenic fungi.

The above disadvantages are also common to the use of synthetic agents manufactured against other pathogens, parasites or pests such as insects, or specific insects, bacteria or specific bacteria and other eukaryotic organisms including, but not limited to: protozoa such as amoebas, intestinal flagellates and ciliates, haemoflagellates, such as leishmania or trypanosomes, sporozoa, such as those responsible for malaria, arthropod-borne organisms; helminths such as trematodes or flukes, cestoidea, acanthocephala, nematodes, trichuris, trichinella, hook worms, filariae, spiruroids; arthropods such as acarina or mites, ticks heteroptera, lice, flees, diptera such as disease-carrying flies including mosquitos, maggots and myiasis.

Inhibition of proteinases is known to occur naturally following pathogen infection. For example, it has been shown that following infection by *Phytophthora infestans* varieties of tomato able to resist the fungus show increased levels of proteinase inhibitors **(1).** This relationship between resistance and the capacity to produce proteinase inhibitors has been used to good effect in the control of pathogen, pests and parasitic diseases. For example, in the most relevant prior art known to the applicant, plant pests are controlled by recombinantly introducing a proteinase inhibitor, animal-derived egg white cystatin, into a selected monocotyledon such as a cereal, forage or turf grass, or a dicotyledon such as a vegetable, tube, or sugar crop, (EP 0 348 348). Similarly, plant nematode pests have been controlled using a proteinase inhibitor, plant-derived cowpea trypsin inhibitor, which has been recombinantly introduced into tobacco, tomato, cotton, oilseed rape, vegetable crop or ornamental plants (EP O 502 730). In addition, it has been suggested that proteinase inhibitors can be used as anti-parasitic proteins which ideally can be administered to a host species either in a medicament or a food (UK Patent Application No. 94 03819.7).

It is therefore known to use proteinase inhibitors to neutralise the effects of proteinases and so combat the effects of pathogens, parasites or pests. In particular, it is known to transgenically produce plants which are provided with a specific proteinase inhibitor, such as a cysteine proteinase inhibitor.

However, it is the object of the present invention to provide a modified proteinase inhibitor which has greater efficacy than that of its unmodified counterpart or the natural proteinase inhibitor; or alternatively to synthetically manufacture and improve a proteinase inhibitor so as to provide, in one embodiment a hybrid proteinase inhibitor.

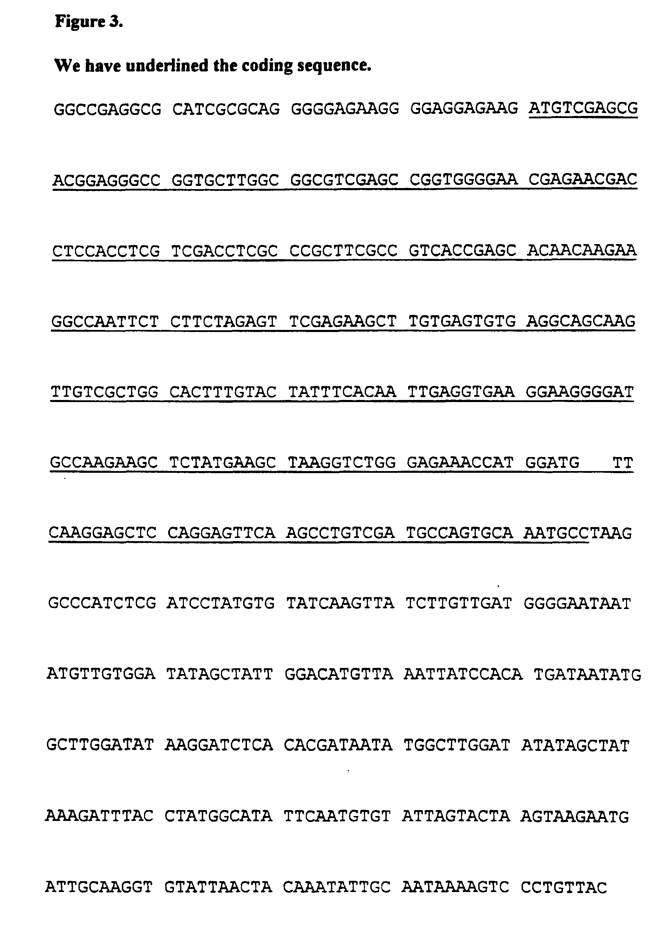

In one aspect of our invention we have focused on the group of proteinase inhibitors known as cystatins. The protein sequences of approximately 25 cystatins are known. It is possible to undertake alignment studies of these sequences in order to provide a basis for identifying structural similarities. It has been suggested that there are sufficient differences between plant and animal cystatins to justify separate classification of the two, indeed, a comparison of a plant cystatin, Oryzacystatin I Oc-I [DNA sequence structure shown in Figure 3], and an animal cystatin, egg white cystatin, reveals a significant number of differences showing that overall amino acid conservation is not high. Moreover, there are significant differences in the binding properties of animal and plant cystatins. Thus the dissociation constant Ki varies, for example, egg white cystatin has a Ki of 5x10⁻¹²M, whereas the plant cystatin, Oc-I, (derived from rice), has a Ki of 3 x 10⁻⁸M.

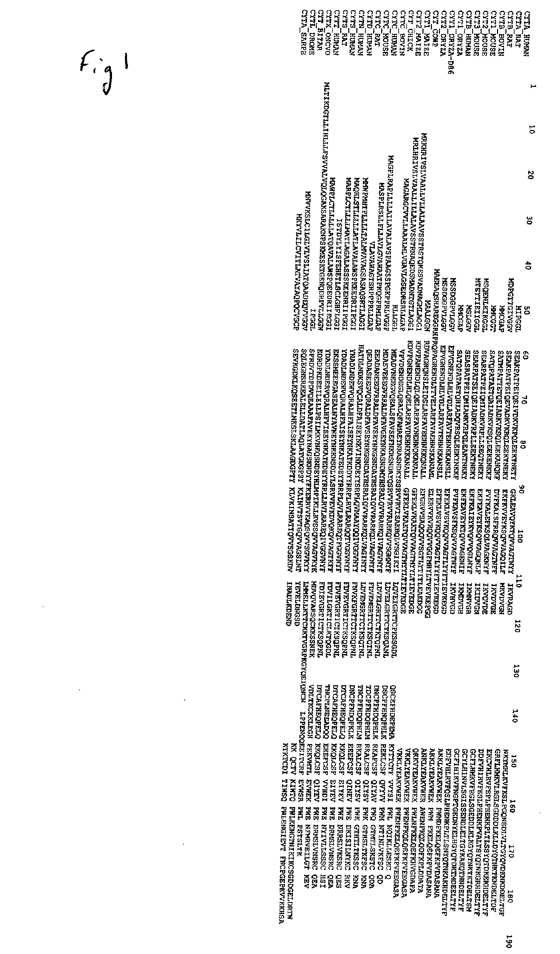

Alignment data of a number of cystatins is shown in Figure 1. The amino acids are numbered 1-181. It can be seen that there is a conserved inhibitory site at alignment amino acids 100-104, represented by the motif QVVAG (or QLVAG). In addition, it can be seen that there is a conserved PW motif at alignment amino acids 160-161.

This conservation occurs in approximately two thirds of the known sequence structures and is thought from structural studies to be involved in the functioning of the protein and thus for inhibition of proteinases. However, some cystatins with low Ki values do not possess this PW motif therefore its importance in cystatin function is unclear.

Other works have recombinantly manufactured novel cystatins. For example, the human cysteine proteinase inhibitor cystatin C, which participates in the intracellular catabolism of proteins and peptides, in the proteolytic conversion of prohormones, in the extracellular degregation of collagen and in the penetration of normal tissues with malignant cells, has been altered. Workers have modified cystatin C so that one or more amino acids at positions 5-17, 55-59 and/or 68 have been replaced by other amino acids thus retaining the total 120 amino acids in the sequence structure. Modifications were undertaken in order to provide an animal-derived cystatin C considered to have constant activity. **(WO 88/09384).**

We have found, surprisingly, that site-directed modification of a plant cystatin such as, for example, Oryzacystatin I (Oc-I) can improve its binding properties and thus improve the efficacy of the enzyme in inhibiting proteinases. The site-directed modification involves elimination of the amino acid aspartic acid at position 86 of the amino acid sequence structure of the plant cystatin, this elimination improves the Ki 13 fold, that is to 2.3 x 10⁻⁹M. This modification is represented by elimination of aspartic acid (symbol D) at position 163 of the alignment amino acids shown in Figure 1.

Clearly, this improvement in plant cystatin Ki does not exceed animal cystatin Ki, and particularly egg white cystatin. However, there is growing concern about the liberal approach to cross species transgenics. That is to say the introduction into one species of genes wholly from another species, such as for example, the introduction into plants of human genes encoding human proteins and visa versa. Until our understanding of the consequences of genetic manipulation is complete is would seem prudent to err on the side of caution and thus adopt a more rational approach to genetic manipulation. Thus, plant breeders throughout the world would prefer to combat plant diseases using plant derived proteins, or alternatively, proteins which are significantly similar to plant proteins such that their structural, biochemical and physiological functions are either the same as, or substantially similar to, or consistent with, that of plant proteins. This letter category includes, but is not limited to hybrid molecules.

Further, many animal cystatins with lower Ki's have several disulphide bonds which are not found in plant cystatins so far characterised. Therefore in order to ensure correct protein folding it may be prudent to use at least partially a plant cystatin in plant systems. Thus, our site-directed modification of a plant proteinase inhibitor, and in particular a plant cystatin, and our hybrid proteinase inhibitor when including at least a part of a plant proteinase inhibitor have provided novel improved proteins for preferred, but not exclusive, use in plant systems.

It is of note that there is very little conservation in the alignment amino acid sequences shown in Figure 1 above alignment amino acid 104 and therefore this makes our observation all the more startling. Previous site-directed modification studies of cystatins were concentrated on the highly conserved QVVAG motif referred to above, but modification of this region was always detrimental (12).

It follows from the foregoing that it is an object of the invention to provide a novel proteinase inhibitor having improved efficacy at least in terms of its binding to a proteinase.

It is also a further object of the invention to provide a novel proteinase inhibitor, ideally having improved efficacy, but also comprising a hybrid molecule which preferably, but not exclusively, comprises a part of a proteinase inhibitor from a first species and a part of proteinase inhibitor from a second species.

It is a further object of the invention to provide products including the whole or a part of the novel protein, or the whole or a part of the DNA encoding same, and also uses for this novel protein and/or DNA.

According to a first aspect of the invention there is therefore provided a proteinase inhibitor modified or manufactured so that it is more effective at inhibiting a proteinase with which it interacts than its corresponding naturally occurring counterpart.

Ideally, said proteinase inhibitor binds more strongly to the proteinase.

Accordingly there is provided a synthetic proteinase inhibitor which has a Ki with at least a 10 times lower value than its natural counterpart. For instance a change in Ki from 3x10⁻⁸M to at least 3x10⁻⁹M represents such an improvement in Ki.

According to a second aspect of the invention there is provided a proteinase inhibitor including at least one site-directed amino acid deletion and/or substitution which lowers the Ki of the protein at least 10 fold.

Preferably the protein is a cystatin and ideally the site-directed deletion or substitution concerns deletion of either aspartic acid at position 86 of the amino acid sequence structure of Oc-I, or alternatively, deletion of aspartic acid at position 163 of the alignment amino acid sequence structure shown in Figure 1 of an aligned proteinase inhibitor such as a cystatin, or alternatively, deletion of its functional counterpart in related proteinase inhibitors or cystatins.

Alternatively, the site-directed modification concerns substitution of said aspartic acid at said position for an alternative amino acid which has counter properties having regard to the functional property of the eliminated aspartic acid.

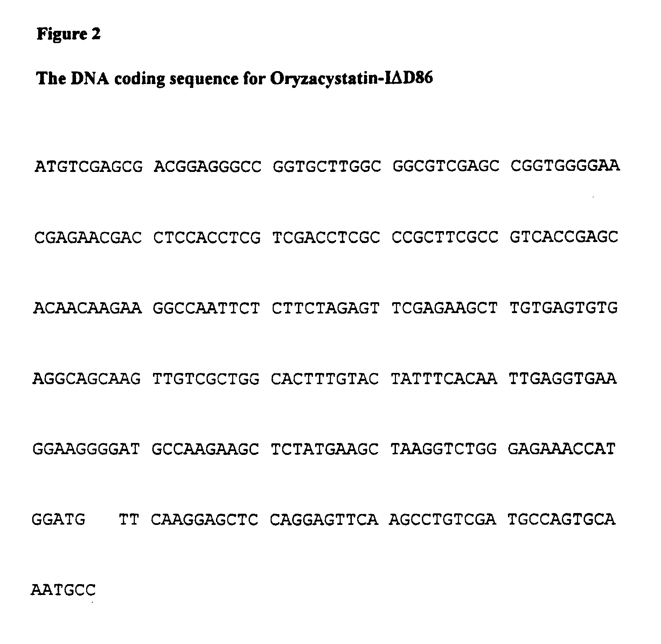

According to a third aspect of the invention there is provided the whole or part of the DNA sequence structure shown in Figure 2 which DNA sequence structure encodes an example of a protein according to a first aspect of the invention.

All the proteins of the invention, or the corresponding DNA sequence structures have utility in combating diseases whose symptoms are at least partially caused by or characterised by proteinase production. Thus the novel proteins and corresponding DNA sequence structures can be used, directly or indirectly, to prevent, alleviate or mitigate such diseased conditions. For example, a host organism suffering from a pathogenic, pest or parasitic condition involving protein breakdown via proteinases can be treated by receiving at least one protein of the invention. Treatment can be undertaken by applying the said protein of the invention directly to the diseased organism, for example, in the form of a chemical agent such as a pesticide, fungicide etc. or a medicament, or alternatively, by introducing the genetic sequence structure for the said protein of the invention into the genome of the host organism and ensuring that the said inventive protein is expressed by the host organism which organism is then equipped to fight the disease.

In the foregoing paragraph any one or more of the proteins of the invention may be used as aforedescribed. For example, a selected combination of the proteins of the invention, that is to say proteins including site-directed modifications and/or hybrid proteins may be used to counter the effects of any one or more proteinases.

Ideally the transformed organism is a plant.

According to a further aspect of the invention there is provided a method of conferring resistance to proteolytic damage comprising modifying or transforming a host organism so that it expresses the protein of the invention.

According to a yet further aspect of the invention there is provided a construct including a whole or part of the DNA sequence structure of the invention. Said construct may include a plasmid or a vector.

According to a yet further aspect of the invention there is provided use of the protein or DNA sequence structure of the invention as a medicament to combat proteolytic conditions ideally of a pathogenic, parasitic or pest nature.

According to a further aspect of the invention there is provided a composition effective against pathogenic, parasitic or pest diseases including the protein of the invention.

According to a yet further aspect of the invention there is provided a transgenic plant transformed with DNA encoding the protein of the invention, and ideally the DNA shown in Figure 2, which DNA is coupled to a suitable promoter sequence so that the protein of the invention can be expressed. Ideally, expression is either generally within the plant or in the locale of the pest, pathogen or parasite interaction with the plant. As examples reference 13 provides general methods for identifying promoters from the locale of a pest, pathogen or parasite of a plant. Alternatively, or in addition expression may be selected so as to occur at a selected given point in time.

Preferably said transformed plant is a cereal crop, vegetable crop, oil crop, sugar crop, forage or turf grass, fibre plant, herbalspice plant, fruit crop or indeed any decorative plant.

According to a yet further aspect of the invention there is provided a transformed organism, plant or otherwise, which includes DNA encoding the protein of the invention, and ideally the DNA shown in Figure 2, so that said protein can be harvested for the purpose of providing sources thereof.

Preferably, said construct is provided with suitable promoters for ensuring expression of the protein of the invention.

According to a yet further aspect of the invention there is provided a method for controlling a pathogen, parasite or pest comprising exposing said pathogen, parasite or pest to the protein of the invention.

According to a further aspect of the invention there is provided use of the protein of the invention to control a pathogen, parasite or pest.

According to a yet further aspect of the invention there is provided any one or more of the primers shown in Table 2, or primers of similar nature having additions, deletions or modifications thereto which still enable the primers to function as described herein.

The modified proteinase inhibitors of the invention may also include novel combinations of proteinase inhibitors either derived from the same or different kingdom, phylum, class, order, family, genus or species. For example, fraction(s) of animal-derived proteinase inhibitor may be combined with fraction(s) of plant-derived proteinase inhibitor, all or one or more of which may or may not include the aforedescribed modification to improve efficacy. Or alternatively, different sorts or types of plant proteinase inhibitors may be combined to provide a novel plant proteinase inhibitor, or alternatively, different sorts or types of animal proteinase inhibitors may be combined to provide a novel proteinase inhibitor, all or one of more of which may or may not include the aforedescribed modification to improve efficacy.

According to a yet further still aspect of the invention there is provided a protein and/or sequence of DNA comprising a first part from a first proteinase inhibitor and at least one other part from at least one other proteinase inhibitor.

In a preferred embodiment of the invention the DNA sequence of the further still aspect of the invention is provided in a construct so that a corresponding protein can be produced in target tissue such as host cell tissue.

According to a yet further aspect of the invention there is provided target tissue or host cell tissue transformed with the DNA sequence structure of the further still aspect of the invention.

According to a yet further aspect of the invention there is provided a protein comprising a first part from a first cystatin and at least one other part from at least one other cystatin.

In a preferred embodiment said first part of said DNA sequence or said protein comprises plant-derived cystatin DNA or protein respectively, and said at least one other part comprises animal-derived cystatin DNA or protein respectively.

Ideally said animal-derived DNA or protein corresponds to DNA or protein from the active site of animal-derived cystatin; and preferably said plant-derived cystatin DNA or protein corresponds to DNA or protein from a structural site or structural sites or said plant-derived cystatins.

Alternatively, said DNA sequence or protein comprises different sorts or types of plant-derived cystatins.

Alternatively again, said DNA sequence or protein comprises different sorts or types of animal-derived cystatin.

According to a yet further aspect of the invention there is provided protein and/or DNA sequence structure relating to a novel proteinase inhibitor comprising both the aforementioned hybrid proteinase inhibitor and also the aforementioned site-directed modification.

All of the proteinase inhibitors of the invention have application for countering the effects of proteinases and for use in methods relating to such effects.

Thus generally speaking the invention relates to the re-design of proteins which exhibit improved functional activities. Site-directed modifications or regions of amino acid sequence are replaced with either a corresponding region of a protein (from any organism) which exhibits the desired characteristics, or with designed synthetic sequences. The amino acid framework of the original protein is ideally maintained in the final hybrid molecule.

The invention will now be described by way of example only with reference to the following figures wherein:
Figure 1 shows the alignment sequence structure of a number of cystatins.
Figure 2 shows the DNA sequence structure of the novel protein of the invention.
Figure 3 shows the DNA sequence structure of the rice cysteine proteinase inhibitor Oryzacystatin Oc-I.
Figure 4 shows the effect of cystatin expression on growth of *G.pallida* females parasitising *A. rhizogenes*-transformed tomato roots. Body size of the nematode is given as the area of its outline in sq µm; **a** controls, **b and c,**Oc-I and Oc-IdeltaD86 expression respectively.
Figure 5 shows the suppression of growth of fungi from spores over 6 days on agar after addition of a total of 45 µgPI(s) to the central well.
Figure 6 shows the relative percentage inhibition of various hybrid proteinase inhibitors when exposed to their corresponding proteinases.
Table 1 shows the dissociation constant Ki of a variety of cystatins either in their native state or when subjected to specific modification.
Table 2 shows the sequence of 23 oligonucleotide primers (P1-23) used in PCR reactions and 2 linkers (L1-2) used in cloning.
Table 2a shows the sequences of primers (P24-39) used in PCR reactions to manufacture hybrid molecules.
Table 3 shows the nature of hybrid molecules manufactured. The bars indicate the length of Oc-I protein, areas shaded black represent the regions replaced by CEWC amino acid sequence.

### Materials and Methods

### Strains and vectors

### DNA preparation and manipulation

Plasmid DNA was purified from *E-coli* cultures by the alkaline lysis method **(2)**. Restriction digests and ligation reactions were carried out using the recommendations of the manufacturer. DNA fragments were recovered from agarose gels using an electroelution chamber (IBI) following the manufacturer's protocol. Oligonucleotides were synthesised on an Applied Biosystems 381A instrument and further purification was only performed for oligonucleotides used in "Altered Sites II" site directed mutagenesis protocols by using a reverse phase COP chromatography cartridge (Cruachem. Glasgow, UK). DNA sequencing of double stranded plasmid DNA was performed using Sequenase version 2.0 (Amersham) according to the manufacturer's instructions.

### Cloning of cystatins and C.elegans proteinase inhibitor

Oc-I was amplified from genomic DNA of *Oryza sativa L. japanica* with primers P1 and P2 (see Table 2) designed from published sequence data **(3)** and with the addition of restriction enzyme sites to assist cloning. The intron was removed by the PCR technique of gene SOEing **(4a & 4b)** where primer pairs P1/P3 and P2/P4 were used to amplify the two exons. These products were then SOEn together by amplifying with primers P1 and P4 and the product cloned into *Sma I*/*Eco* R1 digested pBluescript. The sequence of the cloned coding region was verified by comparing with the published data for *Oc-I* (3). Amplification and intron removal of the *C.elegans* cysteine protease gene, gcp-I, were performed in a similar manner using primers P5-8 (Table 2) designed from sequence information **(5).** The final PCR product was cloned into pBluescript and checked by sequence analysis.

DNA sequence information for cowpea cysteine protease inhibitor, CCPI **(6)** was used to design oligonucleotide primers, P9 and P10 (Table 2). These primers together with a cDNA clone carrying the CCPI gene (kindly provided by Prof. P. Shewry) were used to PCR amplify a product that was cloned directly into the expression vector pQE30 (Qiagen, California, USA) utilising the Bam HI and Hind III sites incorporated into the PCR primers. Genes were cloned into the Type IV pQE expression vectors (Qiagen, California, USA) (Bam HI/Hind III) and proteins were expressed in the *E-coli* strain M15[pREP4].

### Mutagenesis

### a) N-terminal deletions

To generate the large 72bp deletion at the 5'-end of Oc-I, designated pdelta24Oc-I, pQE30/Oc-I was digested with Sma I and Hinc II, the large fragment purified from an agarose gel and relegated. To create the 63bp deletion (termed pdelta21Oc-I), pQE30/Oc-I was restricted with Bam HI and Hinc II and gel purified. The 9bp region immediately 5' to the Hinc II site together with the sequence encoding the enterokinase recognition site was reintroduced by ligating the annealed oligonucleotide linkers L1 and L2 to the purified fragment.

### b) C-terminal deletions

Exonuclease III/mung bean nuclease deletions were carried out **(2)** to generate deletions to 24, 27, 30 and 33bp at the 3'-end of the gene.

### c) Point mutations

The "Unique Site Elimination" (USE) strategy (Pharmacia, Upsalla Sweden) was used to generate constructs expressing single codon alternations using primers P11-P20 (Table 2) which resulted in variant forms of Oc-I having the following amino acid alterations. i) insertion of Leu between 81 and 82. ii) deletion of E13; D86; A74; M85; iii) substitution of (from, No, to) D86N; E89L; Q91L; P83A; W84A. "Altered Sites II" system (Promega, Madison, USA) which involved subcloning Oc-I into the vector pALT-Ex2, (Promega, Madison, USA) was used to generate mutants in which the codons for P83, W84 and D86 were changed to the Amber stop codon (TAG). The oligonucleotides (P21-P23) used to achieve this as shown in Table 2 where the amber stop codons are shown in bold and the point mutation to remove a Sac I site (GAGCTC) is in lower case (this change does not alter the amino acid sequence). The absence of this Sac I site was used as a diagnostic test for mutants. The "Interchange" method (Promega, Madison, USA) was to used to generate amino acid changes, to Cys, Glu, Phe, Gly, His, Pro, Arg, Lys, Gln, Ser and Tyr, at the Amber stop codon by introducing the mutant clones into twelve specific amber suppressing strains.

### Expression of Oc-I and gcp-1 in E coli

Oc-I expressed from pQE30 ("QIAexpression" system) contained six N-terminal histidine residues, encoded by the vector to allow one-step Nickel chelate affinity. Oc-I protein was purified from 11 cultures of *E coli* M15 [pREP4] harbouring the pQE30 derived expression plasmid. 20ml of. an overnight culture was inoculated into 1 litre of LB-media and grown at 37°C to A₆₀₀ 0.7-0.9. IPTG was added to a final concentration of 2mM and growth was allowed to continue for a further 2 h. The cells were harvested by centrifugation at 10000g for 10 min, resuspended in 12ml of sonication buffer (50mM Na₂HPO₄, 300mM NaCI) and stored at -20° C overnight. The sample was thawed, aliquoted to three 15ml tubes and sonicated on ice in short pulses (3x30 sec). Cell debris was pelleted by centrifugation (10000g) and approximately 0.5-0.75ml of Ni-NTA resin slurry (Qiagen, California, USA) was added to each tube and mixed gently on ice for 1h. The resin was collected (1000g for 1 min) and washed five times with 5ml of wash buffer (50mM Na₂HPO₄pH 6.0, 500mM NaCI, 40mM imidazole at 4°C for 0.5h). Protein was eluted with 1ml of elution buffer (50mM Na₂HPO₄, 300mM NaCI, 100mM EDTA) and the resin was repelleted at 1000g for 1 min and the elution repeated a further two times.

The *C.elegans* proteinase glp-1 was expressed in an identical manner.

Delta2IOc-I contained an enterokinase recognition sequence (Asp-Asp-Asp-Asp-Lys) between the N-terminal six histidines (6x His-tail) and the N-terminal residue of the truncated Oc-I protein. Enterokinase (Boeringer) was used to cleave the 6x His-tail from Delta21Oc-I, which was purified from the 6x His-tail by nickel affinity chromatography. "Centricon 10s" (Amicon) were used according to instructions provided with the product to separate OcI from contaminating enterokinase.

### Determination of Ki's

To determine the Ki of cystatins biochemical assays were performed according to the procedure of Barrett **(7)** and Ki values were calculated as described by Abe et al. **(3)**

### SDS PAGE and western blot analysis

All purified proteins were analysed by SDS-PAGE **(8).** Western blots were performed according to the protocol for "mini protein II" (Biorad, Hertfordshire, UK) using PVDF membrane (Millipore, Massachusetts, USA).

### Antibody production

Polyclonal antibodies against Oc-I were raised in male Wistar rats (6 weeks old). Three interperitoneal injections of 100 ug Oc-I in a final volume of 300ml were given at four week intervals. The first injection was an emulsion of protein and complete Freunds adjuvant in a 1:1 (v/v) ratio and the second and third injection were similar but used incomplete Freunds adjuvant. Ten days after the final injection, blood was collected and allowed to coagulate at 4°C before centrifugation at 5000g for 10 min. The resultant serum was collected and stored in 50% (v/v) glycerol at -70° C. The serum gave optimal results in ELISA at a dilution of 1 in 10000 and recognised both native and denatured Oc-I protein.

### ELISAs

ELISAs were performed to determine the level of expression of cystatins in transgenic roots. Root segments of about 2mm were ground in liquid nitrogen, transferred to a 15ml Falcon tube prior to the addition of 1ml of 0.5 x PBS was added and shaking at 4°C for 15 min to dissolve the soluble protein fraction. Protein was acetone precipitated and the precipitant was resuspended in coating buffer (15mM Na₂HCO₃, 34mM NaHCO₃, pH 9.6). Protein concentration was determined by a standard assay **(14).** Wells of a Maxisorb microtitre plate were coated with 100mg protein for 48 h at 4°C. Plates were blocked with anti-Oc-I antibody (1 in 10,000 dilution). Activity was detected by adding substrate and the absorbance of the samples was measured at 405nm when coloration developed.

Different amounts of Oc-I were added to aliquots of 100mg of total protein extracted from untransformed roots and used concurrently in ELISA assays with unknown samples to provide internal standards over the range of 0-2% Oc-I in total soluble protein.

### Culture of C.elegans

*Caenorhaboditis elegans* was cultured on NGM agar carrying a lawn of E-*coli* OP50 cells as described by Wood **(9).** Populations were maintained for 5 days before an agar plug was inserted into fresh media. When required, cystatins were added to the media at a final concentration of 2.5mg 1-¹ just prior to polymerisation. Single nematodes were transferred from non-supplemented solid agar plates to plates containing Oc-I, DeltaD86 Oc-I or BSA. Where necessary ten replicates were carried out for each treatment.

### Transgenic tomato root culture

Oc-I derivative genes were cloned into the vector pBIN19 and then introduced into *Agrobacterium rhizogenes* strain LBA9402 by electrotransformation for use in transformation of *Lycopersicon esculentum* cv Ailsa Craig by a standard protocol **(15)**. Subsequently roots were grown on 0.5x Murashige and Skoog basal salts mixture supplemented with Gamborgs B5 vitamins and 3% sucrose (w/v) and 0.2% phytagel (w/v) plus 100mg 1-¹ kanamycin, solid medium, during initial selection. Western blots were used to confirm the presence of Oc-I or mutant forms in putatively transformed roots.

### Challenge of roots by Globodera pallida

The J2 were obtained from cysts of *G.pallida* and sterilised extensively before use. The cysts were soaked in running tap water for 2-3 days followed by an overnight soak in 0.1% (v/v) malachite green at room temperature. Cysts were then rinsed for 8h in running tap water prior to soaking overnight at 4°C in an antibiotic cocktail (8mg ml-¹ streptomycin sulphate, 6mg ml-¹ penicillin G, 6.13mg ml-¹ polymycin B, 5mg ml-¹ tetracycline and 1mg ml-¹ amphotericin B). The cysts were then washed in filter-sterilised tap water and set to hatch in filter-sterilised potato root diffusate. The overnight hatch of J2s was counted and sterilised sequentially for 10 min with the following antibiotics; 0.1% streptomycin sulphate, 0.1% penicillin G, 0.1% amphotericin B and 0.1% cetyltrimethylammoniumbromide (Cetavlon). The nematodes were pelleted between treatments by brief microcentrifugation. They were washed extensively in filter sterilised tap water and used immediately. Roots of transformed lines were cultured for 4 weeks before 2cm lengths were transferred to fresh media. After a further 3-4 days, 5ml aliquots containing 35 J2s were pipetted onto each actively growing root approximately 1cm from its tip. A 1cm² piece of sterile GFA filter paper was placed over the area to aid infection and was removed 24 h later.

At harvest infected roots were removed from petri dishes, rinsed in water and placed in 1% (w/v) sodium hypochlorite for 2 min. For early time points, roots were plunged into boiling 0.1% aqueous acid fuchsin for 1 min, rinsed in water and then cleared in acidified glycerol at 60°C overnight to facilitate visualisation of nematodes. At the later time points, nematodes could be visualised without staining and were dissected from the roots. Nematodes were examined under a microscope (DBRM, Leica) at 50-200x magnification and the cross-sectional area was measured using an image analyser (Quantimet 5000C;Leica) attached to the microscope.

### Demonstration of antifungal activity

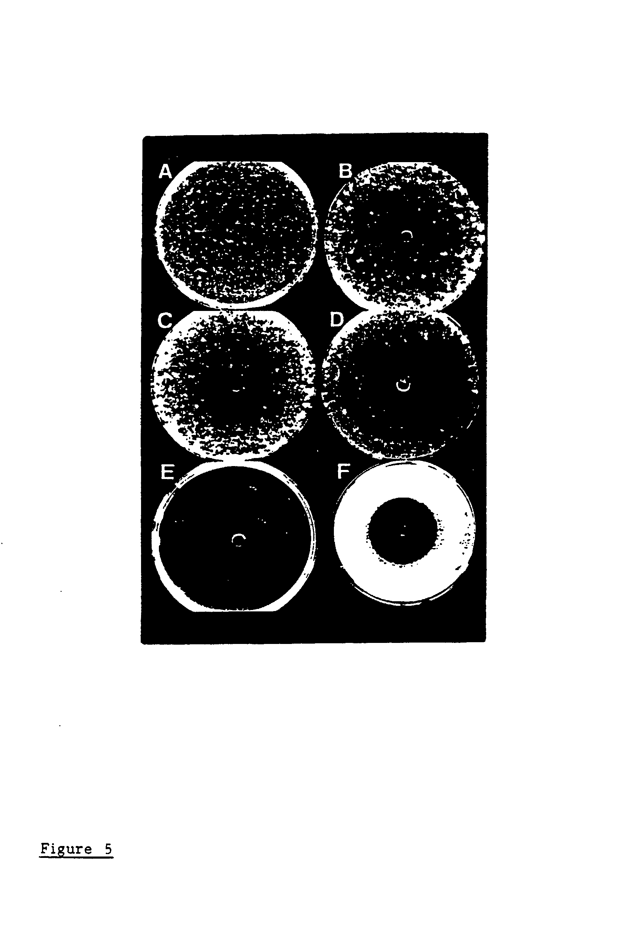

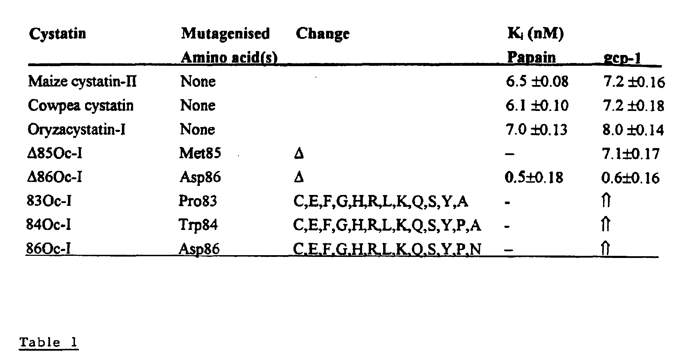

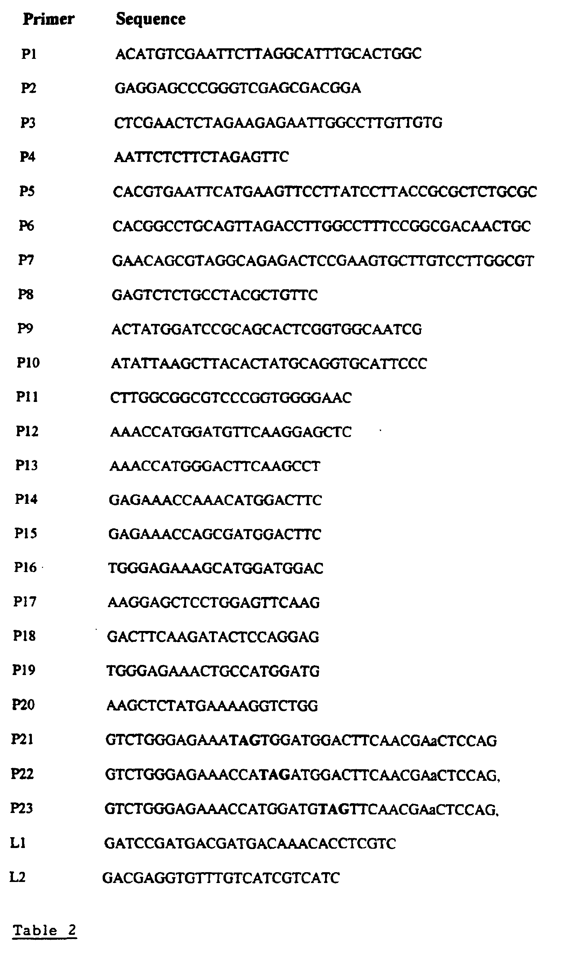

We have shown that the PIs recovered after expression in pQE30 (see earlier) have anti-fungal activity. 45µg of the recovered PI were added at 1 µg/µl to a central well within agar plates which contained spores of *B.cinerea (2.2* x10⁴/plate). The spores do not germinate and the fungus failed to grow where CPTI or Oc-I has diffused into the agar from the central well. The potent effect persisted for many weeks and was enhanced by combining a serine and cysteine PI. Of particular relevance is that Oc-IdeltaD86 was more efficacious than the native form of Oc-I(Figure 3). We have also established that PIs have effects on other micro-organisms including *Aspergillus fumigatus* (a fungal pathogen of mammals; Figure 5) and *Erwinia carotovora* (a bacterial pathogen of plants). This demonstrates two of the central points underpinning this application viz. (i)the approach has a broad potential against very different fungi (ii) protein engineering can enhance the efficacy of PIs against fungi.

### Formation of Hybrid Genes

Chicken egg white cystain (CEWC) is a more potent inhibitor than either Oc-I or Oc-IΔD86. We have replaced fragments of Oc-I with the corresponding sequences of CEWC in order to create a gene of essentially plant origin with the more potent inhibitory properties.

### Materials and Methods

### Replacement of the N-terminus of oc-I with the corresponding region of cewc.

Two oligonucleotide primers (P24 and P25) were synthesised which were overlapping at their 3' ends. These were annealed and filled-in with DNA polymerase I (Klenow fragment) to generate a double-stranded full-length sequence encoding the N-terminus of the mature form of CEWC, **S E D R S R L L G A P V P V D** (residues 1-15, CEWC numbering). Primers P26 and P27 were used to amplify *oc-I* lacking the first 51 bp of coding sequence. The *oc-I* and *cewc* sequences were then joined by a PCR reaction known as SOEing **(4a & 4b**) to generate hybrid gene termed *oc-nterm 1-15*.

### Replacement of the central QWAG and surrounding region of oc-I with the corresponding region of cewc.

A second hybrid molecule was constructed in which the central loop of the tripartite wedge, which comprises the active site of Oc-I, was replaced with the corresponding portion of CEWC. Four oligonucleotides (P28, P29, P30 and P31) were synthesised which when together encoded the CEWC sequence **Y S S R V V R V I S A K R Q L V S G I K Y I L Q** (residues 40-63). Primer pairs P28 / P29 and P30 / P31 were annealed and treated with DNA polymerase I (Klenow fragment) to generate two double-stranded fragments which were subsequently SOEn together. Fragments of *oc-I* encoding both the N- and C-terminal regions were amplified using primers P32 / P33 and P27 / P34 respectively. The synthetic *cewc* sequence (P28-31) was SOEn to the N-terminal fragment of *oc-I* which was in turn SOE-n to the C-terminal fragment of *oc-I* generating the hybrid gene *glvsg40-63*.

### Replacement of the C-terminus of oc-I with the corresponding region of cewc.

The region of *cewc* encoding the C-terminal 20 amino acids, **F V V Y S I P W L N Q I K L L E S K C Q** (residues 97-116) was generated, using primers P35 and P36 in an identical manner to that encoding the N-terminus described above. Primers P32 and P37 were used to amplify *oc-I* lacking the terminal 51 bp of coding sequence. The *oc-I* and *cewc* sequences were SOEn together to generate the hybrid termed *oc-cterm97-116*.

### Generating hybrids with shorter cewc sequences

To determine whether shorter *cewc* regions could confer greater inhibitory potency than native Oc-I in a hybrid cystatin further hybrid molecules containing smaller regions of CEWC were generated. Such hybrid proteins would retain more of the amino acid sequence of the original plant molecule. Two primers P38 and P39, where used in USE mutagenic reactions, this generated two hybrid genes terms *oc-qlvsg52-60* and *oc-pw101-107* which encoded the contiguous *cewc* residues **R Q L V S G I K Y** (residues 52-60 critical *cewc* active site **[12]**) and **S I P W L N Q** (residues 101-107, important region of C-terminus **[16]**) respectively.

### Generating a matrix of hybrid molecules.

Hybrid molecules in which multiple sections of *oc-I* have been replaced by the corresponding *cewc* sequences were generated by utilising conveniently situated restriction enzyme sites in *oc-I.* Sequences encoding the N-terminus of either the native *oc-I* or the N-terminal hybrid gene were removed by *Hinc* II restriction digest. Sequences encoding the C-terminus of either native *oc-I* of the C-terminal hybrid gene were removed by *Foc* I restriction digest. By ligating a fragment from one hybrid gene to that of another, further hybrid molecules were generated in which two or three of the original *oc-I* fragments were replaced by those of *cewc.* A total of 15 hybrid genes were constructed (Table 3).

### RESULTS

### Modelling

Protein sequence alignments were generated using the program SOMAP to align protein sequence selected from the OWL database. An initial alignment of cysteine proteinase sequences served to demonstrate the high degree of conservation throughout the cysteine proteinase family and also confirmed that using papain as our initial target was not unreasonable. A second aligned twenty five cysteine proteinase inhibitors (Figure 1) of available sequences was generated and served to highlight previously identified conserved amino acids and provided a basis for comparing known Ki values of cysteine protease inhibitors with sequence features, information which was used in the design of mutagenesis strategies.

For structural modelling the co-ordinates of hen egg white cystatin **(10)** and human stefin B/papain complex **(11)** were kindly provided by Prof. Bode. These were used to build a three dimensional model of OcI which was then energy minimised using the program Xplor to ensure that our model had reasonable stereochemistry.

### Cloning expression and purification of C.elegans cysteine protease

In order to allow Ki values to be determined against both papain and a nematode protease the *C.elegans* cysteine proteinase was expressed and purified from *E coli* gcp-1 preparations and analysed by SDS PAGE to determine purity.

### Expression and Mutagenesis of cystatins

Cystatins and Oc-I mutational variant proteins expressed and purified from *E coli* were analysed by SDS PAGE to determine the level of purity.

The alignment of the cysteine proteinase inhibitors (Figure 1) shows that Oryzacystatins have no leader sequence (unlike the maize homologues) and are therefore likely to be produced intracellularly. For the three wild type inhibitors, Cowpea protease inhibitor (CCPI), maize cystatin II and Oc-I determination of Ki demonstrated that they have similar inhibitory activities. At the onset of the work CCPI was not characterised. For these reasons Oc-I was selected for mutational analysis.

Previously published reports suggested an N-terminally truncated form of Oc-I was marginally more efficacious than the native protein however, although these variant Oc-I proteins lacked 21 and 24 amino acids of Oc-I they retain N-terminal sequences encoded by vector sequences which might therefore have contributed to interactions with the proteinase. To examine this question proteins were expressed with N-terminal 24 and 21 amino acid deletions and C-terminal 8, 9, 10, 11 and 12 amino acid deletions. The Ki of these variant protein forms were determined using either papain or *C.elegans* protease, gcp-1 (Table 1). None of the truncated proteins were found to have a lower Ki than wild type Oc-I and both 21 and 24 amino acid deletions were inactive, suggesting that the results of other workers were due to the additional vector residues contributing to activity.

Protein sequence alignments and the model of Oc-I together with published Ki values for cystatins were used to identify mutations that might improve the inhibitory capability of Oc-I. The amino acid E13 and D86 were deleted independently. The deletion of residue E13 had no effect on the Ki Oc-I against either papain or gcp-1. Deletion of residue Delta86 lowered the Ki of Oc-I approximately 13-fold from 7nM to 0.5 nm with papain and 8 nM to 0.6 nM with the *C.elegans* protease, gcp-1 (Table 1). The region of the inhibitor around D86 was targeted for further mutagenesis. Table 1 shows Ki values for all the further mutations assayed against the *C*.*elegans* cysteine protease. It is apparent from these figures that all of the substitutional mutations led to an increased Ki suggesting a decreased efficacy. The only mutation which showed a similar or marginally decreased Ki value to that of wild type Oc-I was the deletion of residue M85 (from 8 to 7.1 nM with gcp-1).

### In vivo effect of cystatins against C.elegans

Feeding trials were set up using *C*.*elegans* to examine the effect of protease inhibitors on nematode growth. As soon as hermophrodites became apparent on normal agar they were transferred to individual plates containing either Oc-I, Oc-IdeltaD86, CCPI or BSA and egg laying was observed. Irrespective of the culture media the hermaphrodites laid a mean number of approximately 300 eggs. Half of these eggs were removed to normal plates containing no added inhibitor. The eggs were allowed to hatch and the development of the *C.elegans* larvae was monitored. Under all conditions greater than 95% of the eggs hatched and development was completed for 94%, 92.5%, 97% and 96% of those hatched from eggs recovered from Oc-I, Oc-IdeltaD86, CCPI and BSA supplemented media respectively.

In a second experiment larvae hatching under normal conditions were removed to media supplemented with a protein as above. 50 larvae were transferred at 6 h, 12 h, 24 h and 30 h corresponding to the developmental times when the four larval stages L1, L2, L3 and L4 predominate. No larvae developed to an adult when transferred to media supplemented with Oc-I, Oc-IdeltaD86 or CCPI at 6, 12 or 24 h after hatching. The larvae which failed to develop on cystatin supplemented media became moribund and failed to recover on transfer to fresh non-supplemented plates. These larvae also failed to move or respond to repeated tactile stimuli and eventually died. However 76% of larvae transferred at 30 h after hatching developed to reach the adult male or hermaphrodite stages. All juveniles hatched from eggs laid on media containing BSA and transferred to normal media developed into adult nematodes.

### In vivo effect of cystatin against Globodera pallida

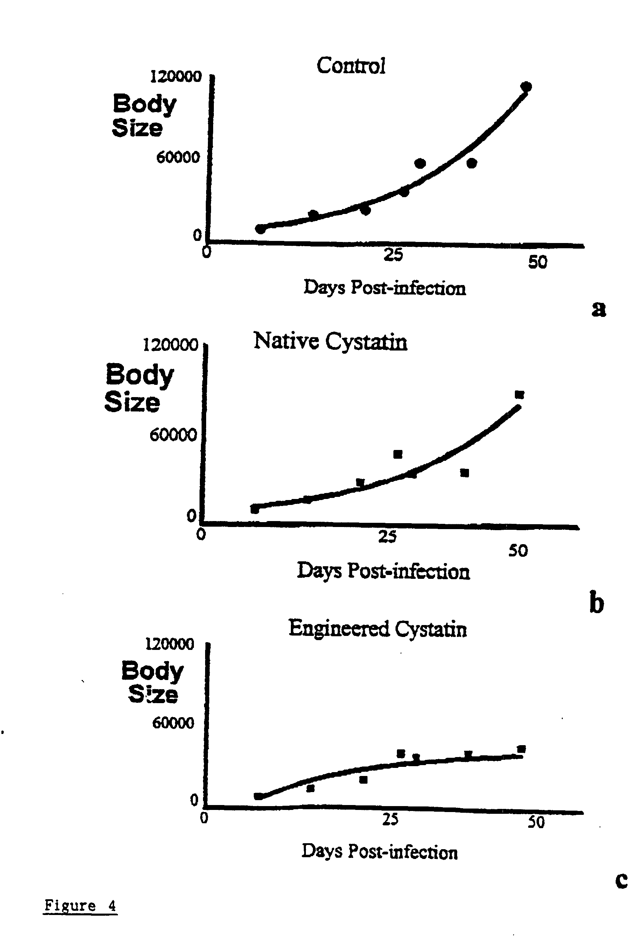

Preliminary assays with Oc-I and Oc-IdeltaD86 demonstrated that anti-Oc-I polyclonal antibodies both recognised both proteins equally well. ELISA established that the highest level of expression in an Oc-I expressing transformed tomato hair root line was 0.54+0.02% of the total soluble protein. Similar assays identified a Oc-IdeltaD86 line with a similar level of expression of 0.51+0.01% of the total soluble protein fraction which was selected for comparative studies. The growth of nematodes on the two transgenic cystatin lines and an untransformed control was measured for individuals recovered from several roots at approximately weekly intervals for 6 weeks. Image analysis provided values for the area of the nematode outline. Means for these values are given against time for three root lines in Figure 4. Statistical analysis was carried out using oneway ANOVA with an *a priori* contrast **(12)** to compare the two cystatin lines against the control for each day of measurement. This analysis establishes a significantly lower outline area (P<0.05) at 1, 2, 4, 5 and 6 weeks. Furthermore no significant increase in size occurred between 4 and 6 weeks for animals on Oc-IdeltaD86 line (P<0.05;SNK) in contrast to the other two lines.

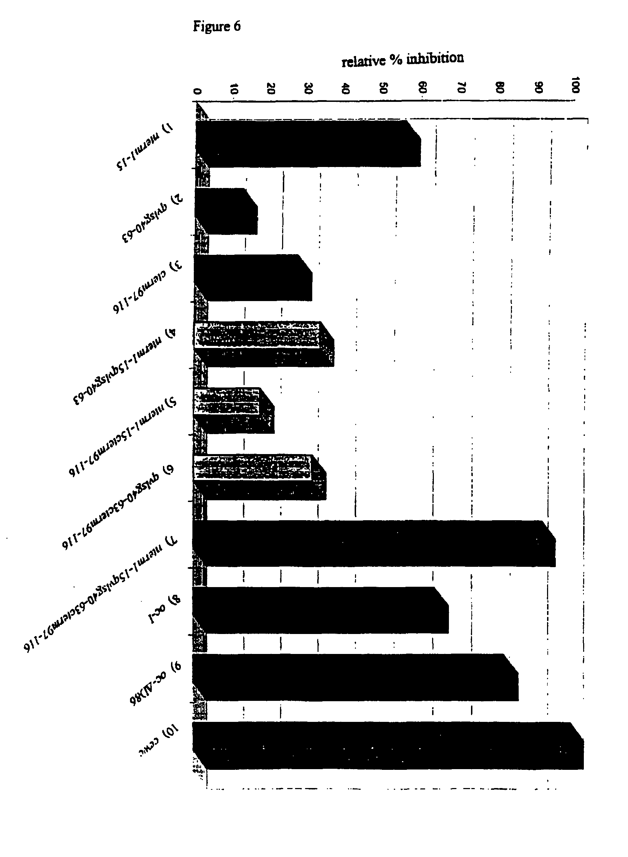

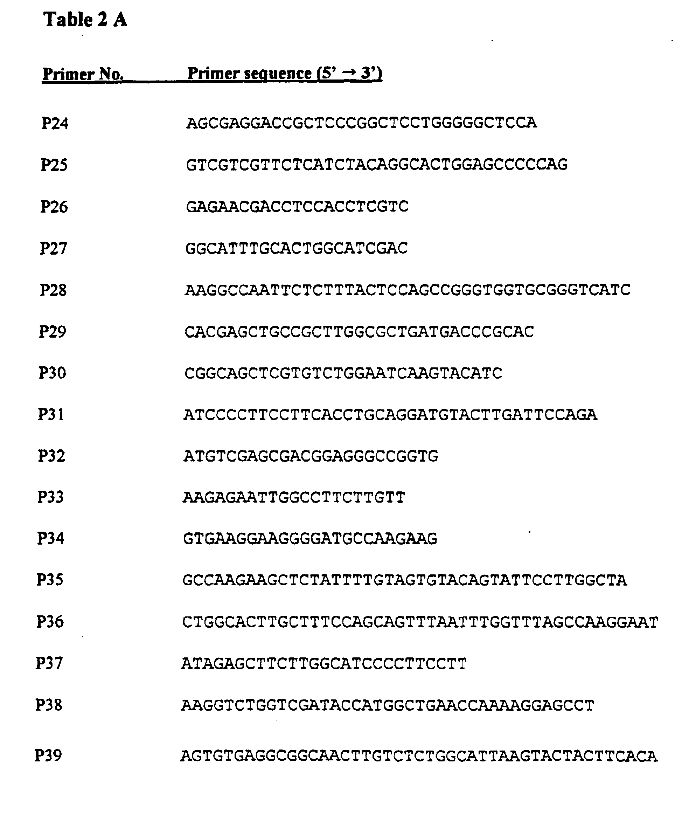

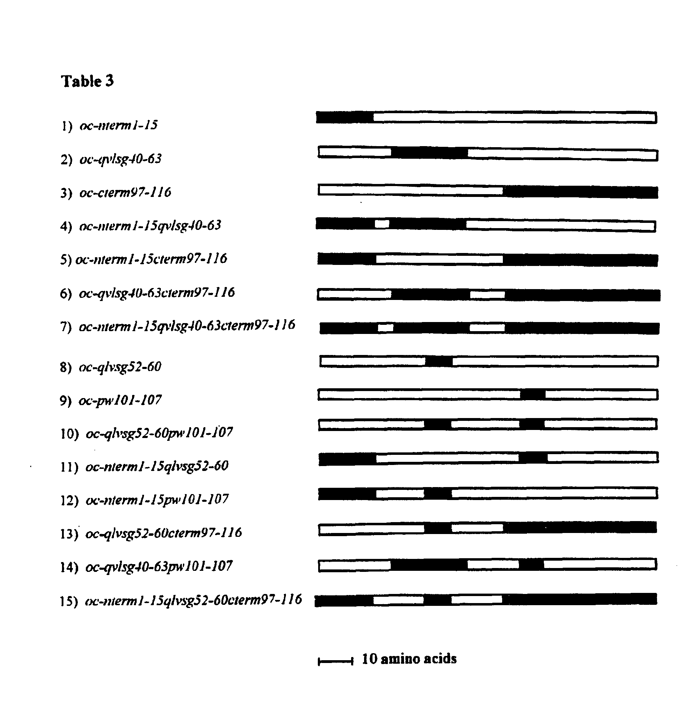

Comparative assays as described by Urwin et al **(16)** in which inhibition by CEWC was determined arbitrarily as 100%, have been carried out the first seven hybrid molecules listed in Table 3. Of those seven only one, OC-NTERM1-15QVLSG40-63CTERM97-116, which contains 57 CEWC residues displayed inhibition of c.a. 90%. This was greater than native Oc-I (c.a. 60%) and Oc-IΔD86 (c.a. 88%). The level of inhibition observed for the remaining six hybrid molecules was reduced (Fig. 6).

### Discussion

It can be seen from the data shown in Table 1 that removal of the aspartic acid at amino acid position 86 improved the Ki value some 13-14 fold whilst deleting methionine at neighbouring position 85 had only a marginal effect on papain inhibition . Additionally, substitution of aspartic acid at position 86 by 12 other amino acids had a detrimental effect on Ki. Therefore, removal of one amino acid, thus shortening the protein backbone, seems to be a significant factor in improving Ki. Moreover, the removal of an amino acid at position 86 seems to be important. We consider that the loop containing this residue is part of the inhibitory site of the molecule with deletion of amino acid aspartic acid 86 resulting in a more similar structure to that of other cystatins perhaps improving the interaction of the conserved above referred to PW site at amino acid positions 83 and 84 with the proteinase.

In Table 1 it can be seen that the efficacy of the native and modified proteinase inhibitors was determined having regard to papain and also gcp-1 derived from the bacteria-feeding nematode *C. elegans*.

Moreover, other information presented herein shows that the modified protein is effective at inhibiting proteinases and so functionally active.

In addition, our data relating to hybrid molecules shows that it is possible to engineer proteins and in particular to modify plant proteins to include at least a part of an animal protein so that the functional effectiveness of a proteinase inhibitor is improved, that is to say the functional effectiveness of a plant protein approaches that of an animal protein.

Our data indicates that previous reports of inadequate control of insects at achievable levels of expression of native proteinase inhibitors may be overcome by using protein engineering as demonstrated in the present application. We have shown that protein engineering can lower and so improve Ki values and so reduce the minimum effective protein level that must be expressed in plants for effective plant protection.

### REFERENCES

**1) Peng, JH & Black LL** (1976) *Phytopatholgy*. **66**:958-963.
**2) Maniatis T.,Fritsch EF & Sambrook J** (eds)(1982). Molecular cloning a laboratory manual. *Cold Spring Harbor Laboratory*. pp545
**3) Abe K, Emori Y, Kondo H, Suzuki K & Arai S.** (1987).Molecular cloning of a cysteine proteinase inhibitor of rice (Orozacystatin) Homology with animal cystains and transient expression of the ripening process of rice seeds. *The Journal of Biological Chemistry.* **262:** 16793-16797.
**4a) Ho SN, Hunt HD, Horton RM, Pullen JK and Pease LR** (1989). *Gene* **77**: 51.
**4b) Horton RM, Hunt HD, Ho SN, Pullen JK and Pease LR** (1989). *Gene* **77**: 61.
**5) Ray C and McKerrow JH** (1992). Gut-specific and developmental expression of a *Caenorhabditis elegans* cysteine protease gene. Molecular and Biochemical Parasitology. **51:** 239-250.
**6) Fernandes KVS, Sabelli PA, Barratt DHP, Richardson M, Xavier-Filho J and Shewry PR** (1993). The resistance of cowpea seeds to bruchid beetles is not related to levels of cysteine proteinase inhibitors. *Plant Molecular Biology* **23**: 215-219.
**7) Barrett AJ** (1972). A new assay for Cathepsin B1 and other thiol proteinases. *Analytical Biochemistry* **47:** 280-293.
**8) Hames BD and Rickwood D**(eds)(1981). Gel electrophoresis of proteins a practical approach. *IRL press limited* pp290.
**9) Wood B** (ed)(1988). The nematode *C.elegans. Cold Spring Harbor Laboratory press*. pp606.
**10) Bode W, Engh R, Musil D, Thiele U, Huber R, Karshikov A,Brzin J,Kos J and Turk V** (1988). The 2.0 Å X-ray crystal structure of chicken egg white cystatin and possible mode of interaction with cysteine proteinases. *The EMBO Journal* 7: 2593-2599.
**11) Stubbs M, Laber B, Bode W, Huber R, Jerala R, Lenarcic B** (1990). The refined 2.4 Å X-ray crystal structure of combinant human stefin B in complex with the cysteine proteinase papain: a novel type of proteinase inhibitor interaction. *The EMBO Journal* **9:** 1939-1947.
**12) Arai S, Watanabe H, Kondo, Emori Y and Abe K** (1991). Papain-inhibitory activity of oryzacystatin, a rice seed cysteine protease inhibitor, depends on the central Gin-Val-Val-Ala-Gly region conserved among cystatin superfamily members. *J Biochem.* **109**:294-298.
**13) Sijmons PC, Atkinson HJ and Wyss U** (1994). Parasitic Strategies of Root Nematodes and Associated Host Cell Responses. *Annual Review of Phytopathology* **32:**235-259.
**14) Bradford M,** (1976). A rapid and sensitive method for the quantitation of microgramme quantities of protein utilising the principle of protein dye binding. *Analytical Biochemistry* **72:**248-254.
**15) Tefer D** (1984). Transformation of several species of higher plant by *Agrobacterium rhizogenes.* Sexual transmition of the transformed genotype and phenotype. *Cell* **37:**959-967.
**16) Urwin PE, Atkinson, HJ, Waller D and McPherson MJ** (1995). Engineered Oryzacystatin-I expressed in transgenic hairy roots confers resistance to *Globodera pallida*. The Plant Journal 8: 121-131.

A proteinase inhibitor modified or manufactured so that it is more effective at inhibiting a proteinase with which it. interacts than its corresponding naturally occurring counterpart.

A proteinase inhibitor according to the invention wherein the inhibitor binds more strongly to the said proteinase.

A proteinase inhibitor according to the invention wherein the inhibitor has a Ki at least ten times lower than its natural counterpart.

A proteinase inhibitor according to the invention including at least one site-directed amino acid deletion.

A proteinase inhibitor according to the invention wherein the deletion concerns deletion of aspartic acid at position 163 of an amino acid sequence structure which is aligned with the alignment amino acid sequence structure shown in Figure 1, or alternatively deletion of its functional counterpart in related proteinase inhibitors.

A proteinase inhibitor according to the invention wherein said inhibitor is a cystatin.

A proteinase inhibitor according to the invention wherein the protein is Oryzacystatin-I(Oc-I) and the deletion is deletion of the aspartic acid at position 86 of this protein.

A proteinase inhibitor according to the invention wherein said deletion is followed by a substitution at said position for an alternative amino acid.

A proteinase inhibitor according to the wherein said alternative amino acid has counter properties having regard to the functional properties of the eliminated aspartic acid.

Use of a proteinase inhibitor according to the invention to counter the effects of a corresponding proteinase.

Use according to the invention wherein the inhibitor is used as a medicament.

Use according to the invention wherein the inhibitor is used as a pesticide.

A composition for use in countering the effects of a proteinase comprising at least a proteinase inhibitor according to the invention

A composition according to the invention which further includes a suitable carrier.

The DNA sequence structure corresponding to a proteinase inhibitor in accordance with the invention

A construct including the DNA sequence structure according to the invention.

An organism transformed with the construct according to the invention

An organism according to the invention which is a plant.

A method of conferring resistance to proteolytic damage comprising modifying or transforming an organism so that it expresses an inhibitor according to the invention.

A method according to the invention whereby said expression is selected so that expression can occur either temporarily or locally so that the inhibitor is either expressed at a given selected point in time or at given location with respect to the physiology and/or anatomy of the organism.

A method according to the invention wherein said organism is a plant.

A method according to the invention wherein said plant is a cereal crop.

A method of producing a proteinase inhibitor according to the invention wherein a host organism is transformed such that its genetic material includes DNA sequence structure relating to a proteinase inhibitor according to the invention and then said transformed organism is cultured and/or cultivated under conditions wherein said proteinase inhibitor is expressed with a view to harvesting same.

A method of controlling a pathogen, parasite or pest comprising exposing said pathogen, parasite or pest to a proteinase inhibitor according to the invention

A method for producing a proteinase inhibitor according to the invention
which comprises the use of any one or more of the primers shown in Table 2, or primers of similar nature having additions, deletions or modifications thereto which still enables the primer to produce a modified proteinase inhibitor as herein described.

Primers as herein disclosed

A hybrid proteinase inhibitor comprising a first part corresponding to a first proteinase inhibitor and at least one other part corresponding to at least one other proteinase inhibitor.

A proteinase inhibitor according to the invention wherein at least one of said parts corresponds to a cystatin.

A proteinase inhibitor according to the invention wherein at least a second part corresponds to a cystatin.

A proteinase inhibitor according to the invention wherein at least one cystatin is of plant origin.

A proteinase inhibitor according to the invention wherein said first part corresponds to a plant cystatin and said at least one other part corresponds to an animal cystatin.

A proteinase inhibitor according to the invention wherein said plant cystatin part corresponds to the structural portion of said corresponding plant cystatin.

A proteinase inhibitor according to the invention wherein said animal cystatin corresponds to an active portion of the corresponding animal cystatin.

A proteinase inhibitor according to the invention wherein said inhibitor comprises a multitude of parts, each part corresponding to a proteinase inhibitor and the nature of each part selected so as to optimise the functional characteristics of the hybrid molecule.

A proteinase inhibitor according to the invention wherein a first part corresponds to least a part of a proteinase inhibitor according to the invention at least a second part corresponds to at least a part of proteinase inhibitor according to the invention.

DNA sequence structure corresponding to the proteinase inhibitor according to the invention

A construct including the DNA sequence structure according to the

A host cell transformed with either the DNA sequence structure according to the and/or the construct according to the invention.

Use of a proteinase inhibitor according to the invention to counter the effects of a corresponding proteinase.

Use according to the invention wherein the inhibitor is used as a medicament.

Use according to the invention wherein the inhibitor is used as a pesticide.

A composition for use in countering the effects of a proteinase comprising at least a proteinase inhibitor according to the invention.

A composition according to the invention which further includes a suitable carrier.

A method of conferring resistance to proteolytic damage comprising modifying or transforming an organism so that it expresses an inhibitor according to the invention

A method according to the invention whereby said expression is selected so that expression can occur either temporary or locally so that the inhibitor is either expressed at a given selected point in time or at a given location with respect to the physiology and/or anatomy of the organism.

A method according to the invention wherein said organism is a plant.

A method of producing a proteinase inhibitor according to the invention wherein a host organism is transformed such that its genic material includes DNA sequence structure according to the invention and then said transformed organism is cultured and/or cultivated under conditions wherein said proteinase inhibitor is expressed with a view to harvesting same.

A method of controlling a pathogen, parasite or pest comprising exposing said pathogen, parasite or pest to a proteinase inhibitor according to the invention.

A method of producing a proteinase inhibitor according to the invention which comprises the use of any one or more of the primers should in Table 2a, or primers of similar nature having additions, deletions or modifications thereto which still enables the primer to produce a modified proteinase inhibitor as herein described.

## Claims

1. A hybrid proteinase inhibitor comprising:
(i) a first part derived from a first cystatin; and
(ii) a second part derived from one or more cystatins wherein said second part is not found in said first cystatin **characterised in that** said hybrid proteinase is more effective at inhibiting a protease with which it interacts.

2. A hybrid proteinase inhibitor comprising:
(i) a first part derived from a first cystatin;
(ii) a second part derived from one or more cystatins wherein said second part is not found in said first cystatin;
**characterised in that** at least one part includes at least one site-directed amino acid deletion, wherein the deletion is of aspartic acid at position 86 of oryzacystatin I, or of an equivalent residue in a homolgue or analogue thereof following alignment of the protein sequence structure of said homolgue or analogue with the protein sequence structure of oryzacystatin I.

3. A hybrid proteinase inhibitor according to claim 1 or 2 wherein the first part consists of a plant cystatin and the second part consists of an animal cystatin.

4. A hybrid proteinase inhibitor according to claim 3 consisting of a plant cystatin wherein at least one of the N-terminus, the C-terminus and the active site are wholly or partially replaced with equivalent regions from an animal cystatin.

5. A hybrid proteinase inhibitor according to claim 3 or 4, wherein the animal cystatin is chicken egg white cystatin.

6. A hybrid proteinase inhibitor according to claim 3 or 4, wherein the plant cystatin is oryzacystatin.

7. A DNA molecule encoding the proteinase inhibitor according to any one of claims 1 to 6.

8. A construct including the DNA sequence structure according to claim 7.

9. A construct according to claim 8 wherein said construct is a vector.

10. A non-human host cell transformed with a DNA molecule according to claim 7 and/or a construct according to claim 8 or 9.

11. A cell according to Claim 10 wherein said cell is a plant cell.

12. A seed comprising a plant cell according to Claim 11.

13. A proteinase inhibitor according to any of claims 1 to 6, for use as a pesticide.

14. A proteinase inhibitor according to any of claims 1 to 6 for use in medicine.

15. The use of proteinase inhibitor according to any of claims 1 to 6 in the manufacture of a medicament for use in the inhibition of a corresponding proteinase.

16. A composition for countering the effects of a proteinase including at least one proteinase inhibitor according to any one of claims 1 to 6.

17. A composition according to claim 16 which further includes a suitable carrier.

18. A method of conferring resistance to proteolytic damage including modifying or transforming a plant so that it expresses an inhibitor according to any one of claims 1 to 6.

19. A method according to claim 18 wherein said expression is governed by a selected promoter isolated from a suitable gene or modified so that expression can occur either temporarily or locally so that the inhibitor is either expressed at a given selected point in time or at a given location with respect to the physiology and/or anatomy of the organism.

20. A method of producing a proteinase inhibitor according to any of claims 1 to 6 wherein a non-human host organism is transformed such that its genetic material includes a DNA molecule encoding a proteinase inhibitor according to any one of claims 7-9 and then said transformed organism is cultured and/or cultivated under conditions wherein said proteinase inhibitor is expressed with a view to harvesting the same.

21. A method of controlling a pathogen, parasite or pest including exposing said pathogen, parasite or pest to a proteinase inhibitor according to any one of claims 1 to 6.

22. A method of producing a proteinase inhibitor according to any one of claims 1 to 6 which includes the use of any one or more of the primers shown in Table 2a, or primers having additions, deletions or modifications thereto which still enables the primer to produce said proteinase inhibitor.
